(19) 

Europäisches Patentamt
European Patent Office
Office européen des brevets

(11)  **EP 4 129 963 A1**

(12)  **EUROPEAN PATENT APPLICATION**
published in accordance with Art. 153(4) EPC

(43) Date of publication:
08.02.2023 Bulletin 2023/06

(21) Application number: 21780012.7

(22) Date of filing: 26.03.2021

(51) International Patent Classification (IPC):
$C07C\ 1/24^{(1968.09)}$     $B01J\ 23/06^{(1974.07)}$
$B01J\ 23/34^{(1974.07)}$     $B01J\ 23/75^{(1995.01)}$
$B01J\ 23/755^{(1995.01)}$     $C07C\ 11/09^{(1980.01)}$
$C07C\ 51/235^{(1980.01)}$     $C07C\ 51/25^{(1980.01)}$
$C07C\ 57/05^{(1980.01)}$     $C07C\ 67/08^{(1974.07)}$
$C07C\ 69/54^{(1968.09)}$     $C07B\ 61/00^{(1985.01)}$

(52) Cooperative Patent Classification (CPC):
(C-Sets available)
B01J 23/75; B01J 23/06; B01J 23/34; B01J 23/72;
B01J 23/745; B01J 23/755; B01J 37/0236;
C07C 1/24; C07C 11/09; C07C 29/04; C07C 45/28;
C07C 51/235; C07C 51/25; C07C 57/04;
C07C 67/08;             (Cont.)

(86) International application number:
PCT/JP2021/012965

(87) International publication number:
WO 2021/200689 (07.10.2021 Gazette 2021/40)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2020 JP 2020064548**

(71) Applicant: **Mitsubishi Chemical Corporation**
**Chiyoda-ku**
**Tokyo 100-8251 (JP)**

(72) Inventors:
• SUZUKI Tatsuya
  Tokyo 100-8251 (JP)
• TAKEDA Akio
  Tokyo 100-8251 (JP)
• KATOU Yuuki
  Tokyo 100-8251 (JP)
• NINOMIYA Wataru
  Tokyo 100-8251 (JP)

(74) Representative: **Hoffmann Eitle**
**Patent- und Rechtsanwälte PartmbB**
**Arabellastraße 30**
**81925 München (DE)**

(54) **CATALYST, METHOD FOR PRODUCING ISOBUTYLENE, METHOD FOR PRODUCING METHACRYLIC ACID, AND METHOD FOR PRODUCING METHYL METHACRYLATE**

(57) There is provided a catalyst that enables the production of isobutylene with a high selectivity in the production of isobutylene by dehydration of isobutanol. The catalyst according to the present invention contains at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase.

FIG. 1

EP 4 129 963 A1

**(Cont. next page)**

(52) Cooperative Patent Classification (CPC): (Cont.)
   **C07C 69/54;** B01J 21/04; B01J 35/023;
   C07B 61/00; C07C 2521/04; C07C 2523/06;
   C07C 2523/34; C07C 2523/72; C07C 2523/745;
   C07C 2523/75; C07C 2523/755

   C-Sets
   **C07C 1/24, C07C 11/09;**
   **C07C 29/04, C07C 31/12;**
   **C07C 45/28, C07C 47/22;**
   **C07C 51/235, C07C 57/04;**
   **C07C 51/25, C07C 57/04;**
   **C07C 67/08, C07C 69/54**

**Description**

[Technical Field]

The present invention relates to a catalyst, a method for producing isobutylene, a method for producing methacrylic acid, and a method for producing methyl methacrylate.
Priority is claimed on Japanese Patent Application No. 2020-064548 filed in Japan on March 31, 2020, the contents of which are incorporated herein by reference.

[Background Art]

Isobutylene is one of the important raw materials of chemicals, which is converted to ethyl tert-butyl ether, paraxylene, methyl methacrylate, and the like. For example, isobutylene or tert-butyl alcohol obtained by hydrating isobutylene is subjected to gas phase oxidation to obtain methacrylic acid, and then the obtained methacrylic acid is subjected to esterification with methanol, whereby methyl methacrylate can be obtained.
As a method for producing isobutylene, for example, it is known a method in which isobutanol is brought into contact with a catalyst such as alumina under pressure to dehydrate isobutanol to produce isobutylene (for example, Patent Document 1). However, it is difficult to produce isobutylene with a high selectivity by a method in the related art, such as Patent Document 1.

[Citation List]

[Patent Document]

[Patent Document 1]
PCT International Publication No. WO2015/170686

[Summary of Invention]

[Technical Problem]

An object of the present invention is to provide a catalyst that enables the production of isobutylene with a high selectivity in the production of isobutylene by dehydration of isobutanol, as well as a method for producing isobutylene, in which the above catalyst is used, a method for producing methacrylic acid, and a method for producing methyl methacrylate.

[Solution to Problem]

The present invention has the following configurations.

[1] A catalyst containing at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase.
[2] The catalyst according to [1], in which a content of the metal is 0.025 mmol or more with respect to 1 g of the alumina.
[3] A method for producing isobutylene, including producing isobutylene from isobutanol using the catalyst according to [1] or [2].
[4] A method for producing methacrylic acid, including producing methacrylic acid from the isobutylene produced according to the method for producing isobutylene according to [3].
[5] A method for producing methacrylic acid, including obtaining tert-butyl alcohol from the isobutylene produced according to the method for producing isobutylene according to [3], and subsequently producing methacrylic acid from the obtained tert-butyl alcohol.
[6] A method for producing methyl methacrylate, including producing methyl methacrylate from the methacrylic acid produced according to the method for producing methacrylic acid according to [4] or [5] and from methanol.
[7] A method for producing isobutylene, including producing isobutylene from isobutanol by using a catalyst including at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase.
[8] A method for producing methacrylic acid, including producing isobutylene that is produced according to the method for producing isobutylene according to [7], and producing methacrylic acid from the isobutylene.

[9] A method for producing methacrylic acid, including obtaining tert-butyl alcohol from the isobutylene produced according to the method for producing isobutylene according to [7], and subsequently producing methacrylic acid from the obtained tert-butyl alcohol.

[10] A method for producing methyl methacrylate, including producing methyl methacrylate from the methacrylic acid produced according to the method for producing methacrylic acid according to [8] or [9] and from methanol.

[Advantageous Effects of Invention]

According to the present invention, it is possible to provide a catalyst that enables the production of isobutylene with a high selectivity in the production of isobutylene by dehydration of isobutanol. In addition, according to the present invention, it is possible to provide a method for producing isobutylene, in which the catalyst according to the present invention is used, a method for producing methacrylic acid, and a method for producing methyl methacrylate.

[Brief Description of Drawings]

Fig. 1 is a graph showing the isobutylene selectivity in the C4 gas in Examples 1 to 4 and Comparative Example 1.

Fig. 2 is a graph showing the isobutylene selectivity and WHSV in the C4 gas in Examples 5 to 12 and Comparative Example 2.

Fig. 3 is a graph showing the isobutylene selectivity and WHSV in the C4 gas in Examples 13 to 17 and Comparative Example 3.

Fig. 4 is a graph showing the isobutylene selectivity and WHSV in the C4 butene in Examples 18 to 24.

Fig. 5 is a graph showing the isobutylene selectivity and the isobutanol conversion rate in the C4 gas in Examples 25 to 29 and Comparative Example 4.

[Description of Embodiments]

[Catalyst]

The catalyst according to the present invention is a catalyst for producing isobutylene from isobutanol. The catalyst according to the present invention contains at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase. By using the catalyst according to the present invention, the dehydration of isobutanol via diisobutyl ether is promoted on alumina, and thus isobutylene can be produced with a high selectivity.

The alumina crystal system that is used in the present invention is not particularly limited as long as it contains alumina consisting of one or more crystal phases of a monoclinic crystal phase, a tetragonal crystal phase, and a cubic crystal phase. As the crystal phase, it is possible to use various kinds of alumina such as $\alpha$-alumina, $\beta$-alumina, $\gamma$-alumina, $\alpha$-alumina, $\theta$-alumina, $\delta$-alumina, $\eta$-alumina, and alumina hydrate. In particular, from the viewpoint of activity and selectivity, it is preferably $\gamma$-alumina, $\delta$-alumina, $\theta$-alumina, or $\eta$-alumina, and more preferably $\gamma$-alumina or $\eta$-alumina. Alumina in these crystal forms may be used alone or in a combination of two or more thereof. When two or more kinds thereof are used in combination, those having crystal forms different from each other may be used, or a crystal state of a mixed phase may be taken.

The alumina that is used in the catalyst according to the present invention can be easily produced by a known method including, for example, a thermal decomposition method, a precipitation method, a deposition method, a kneading method, or a method in which these methods are used in combination. Examples of the raw material for alumina include a material that generates alumina or alumina hydrate by heating or hydrolysis, such as a nitrate, an acetate, an alkoxide, a sulfate, a chloride, an alkali aluminate, or alum. Examples of the alkali that is used in hydrolysis include caustic alkali, alkaline carbonate, aqueous ammonia, and ammonium carbonate.

The alumina that is used in the catalyst according to the present invention may contain other compounds. In order to improve the isobutylene selectivity, the purity of alumina is preferably 90.0% by mass or more, more preferably 95.0% by mass or more, still more preferably 97.0% by mass or more, even still more preferably 98.0% by mass or more, particularly preferable, 99.0% by mass or more, and most preferably 99.5% by mass or more.

Examples of the other compounds include $SiO_2$ and $Na_2O$.

The content of $SiO_2$ in alumina is preferably 1.00% by mass or less, more preferably 0.75% by mass or less, still more preferably 0.50% by mass or less, even still more preferably 0.40% by mass or less, even further still more preferably 0.30% by mass or less, and most preferably 0.20% by mass or less, with respect to the total mass of alumina. $SiO_2$ may not be contained in alumina.

The content of $Na_2O$ in alumina is preferably 0.20% by mass or less, more preferably 0.15% by mass or less, still more preferably 0.10% by mass or less, even still preferably 0.075% by mass or less, even still more preferably 0.050% by mass or less, and most preferably 0.025% by mass or less, with respect to the total mass of alumina. $Na_2O$ may not be contained in alumina.

The contents of alumina, $SiO_2$, and $Na_2O$ are measured by an ICP emission spectroscopic analysis (ICP-AES). For example, they can be measured by Optima 8300 ICP-OES Spectrometer manufactured by Perkin Elmer, Inc.

From the viewpoint that sufficient activity can be easily obtained, the BET specific surface area of alumina is preferably 50.0 $m^2/g$ or more, more preferably 60.0 $m^2/g$ or more, still more preferably 70.0 $m^2/g$ or more, particularly preferably 80.0 $m^2/g$ or more, and most preferably 90.0 $m^2/g$ or more. The upper limit of the BET specific surface area of alumina is not particularly limited; however, it is preferably 350 $m^2/g$ or less.

The BET specific surface area of alumina is a value calculated from an $N_2$ adsorption/desorption isotherm, and it can be measured using, for example, Tristar 3000 (product name, manufactured by Shimadzu Corporation).

The metal contained in the catalyst according to the present invention is at least one metal selected from Group 6 to Group 14 metal elements in Period 4 to Period 6 of the periodic table. From the viewpoint that the isobutylene selectivity is high, it is preferably at least one selected from Cr, Mn, Fe, Co, Ni, Cu, Zn, Ga, Ge, Mo, Tc, Ru, Rh, Pd, Ag, Cd, In, and Sn, it is more preferably at least one selected from Cr, Mn, Fe, Co, Ni, Cu, Zn, Ga, Ge, Ru, Rh, Pd, Ag, Cd, and In, it is still more preferably at least one selected from Cr, Mn, Fe, Co, Ni, Cu, Zn, Ga, Ge, Ru, Rh, Pd, and Ag, it is even still more preferably at least one selected from Cr, Mn, Fe, Co, Ni, Cu, Zn, Ga, and Ge, it is particularly preferably at least one selected from Mn, Fe, Co, Ni, Cu, Zn, and Ga, and it is most preferably at least one selected from Mn, Fe, Co, Ni, Cu, and Zn. The metal contained in the catalyst may be one kind or two or more kinds.

The metal content of the catalyst according to the present invention is preferably 0.80 mmol or less, more preferably 0.70 mmol or less, still more preferably 0.60 mmol or less, particularly preferably 0.50 mmol or less, and most preferably 0.40 mmol or less with respect to 1 g of alumina. When the content of the metal in the catalyst is equal to or smaller than the above-described upper limit value, both the catalytic activity and the isobutylene selectivity can be achieved. Further, with respect to 1 g of alumina, it is preferably 0.025 mmol or more, more preferably 0.050 mmol or more, still more preferably 0.075 mmol or more, particularly preferably 0.10 mmol or more, and most preferably 0.15 mmol or more. When the content of the metal in the catalyst is equal to or larger than the above-described lower limit value, the formation of linear butenes can be suppressed and a high isobutylene selectivity can be obtained. The above-described upper and lower limits may be combined in any combination. For example, the metal content of the catalyst according to the present invention is preferably 0.025 mmol or more and 0.80 mmol or less, more preferably 0.050 mmol or more and 0.70 mmol or less, still more preferably 0.075 mmol or more and 0.60 mmol or less, even still more preferably, 0.10 mmol or more and 0.50 mmol or less, and particularly preferably 0.15 mmol or more and 0.40 mmol or less, with respect to 1 g of alumina.

A method for producing the catalyst according to the present invention is not particularly limited, and a known method can be used. For example, the catalyst can be easily produced by a coprecipitation method, a deposition method, an impregnation method, a kneading method, or a method in which these are used in combination. The raw material for the metal contained in the catalyst is not particularly limited, and examples thereof include a hydroxide, a nitrate, an acetate, and a sulfate.

The catalyst according to the present invention may be molded as necessary. For example, in a case of a gas phase fixed bed reaction, it is preferable to determine the shape of the catalyst in consideration of the pressure loss in the reactor and the diffusion of a gas. Further, in both the gas phase fluidized bed reaction and the liquid phase reaction, it is preferable to determine the shape of the catalyst in consideration of the reaction conditions and material transfer.

Examples of the method for molding the catalyst according to the present invention include a method using a powder molding machine such as a tableting molding machine, an extrusion molding machine, or a rolling granulator. As the shape in a case of molding the catalyst according to the present invention, any shape such as a spherical shape, a ring shape, a columnar shape, or a star shape can be adopted. The molded catalyst may be ground and used as a powder. An additive may be mixed with the catalyst at the time of molding.

[Method for producing isobutylene]

The method for producing isobutylene according to the present invention is a method of producing isobutylene from isobutanol using the catalyst according to the present invention.

The isobutanol that is used as a starting raw material is not particularly limited, and it may be biomass-derived isobutanol from the viewpoint of environmental protection. As the starting raw material for isobutanol, fossil raw material-derived isobutanol and biomass-derived isobutanol can be mixed and used.

The "biomass-derived isobutanol" is either isobutanol purified from an organic compound obtained by using fermentable sugar of biomass and subjecting it to a fermentation process or isobutanol obtained by a process including any one or more processes of catalytic chemical conversion and thermochemical conversion of biomass.

The biomass can be broadly divided into one derived from resource crops and one derived from waste. Examples of the biomass derived from resource crops include food crops, wood, and flowers, and unused portions of these crops can also be used. Examples of the biomass derived from waste include food waste, sludge such as sewage, livestock excreta, and waste paper.

Dehydration of isobutanol may be carried out in the liquid phase or in the gas phase. When the reaction is carried out in the gas phase, a type of a fixed bed, a fluidized bed, or the like can be adopted. Hereinafter, a case where the reaction is carried out in the gas phase will be described; however, the present invention is not limited thereto.

For example, by vaporizing a raw material with a vaporizer, it can be supplied to a reactor as a raw material gas. The vaporizer is not particularly limited, and examples thereof include a jacket type, a horizontal tube type with a natural circulation system, an immersion tube type with a natural circulation system, a vertical short tube type with a natural circulation system, a rising membrane type having a vertical long tube, a descending membrane type having a horizontal tube, a horizontal tube type with a forced circulation system, a vertical tube type with a forced circulation system, and a coil type. It is also possible to use a method in which a heating coil is simply wound around a raw material supply pipe, and a raw material moving in the raw material supply pipe is vaporized in the raw material supply pipe before entering a reactor and supplied into the reactor in a gas state. The vaporizer is not particularly limited as well, similarly when a component other than the raw material, such as a diluent gas, is vaporized and supplied into the reactor.

The conditions for vaporizing the raw material are not particularly limited, and for example, the temperature can be 108°C or higher and 600°C or lower, and the pressure can be 0.05 MPa or higher and 1 MPa or lower in terms of absolute pressure.

In the raw material gas, the isobutanol concentration can be adjusted by diluting isobutanol with a diluent gas. The raw material gas may be a gas consisting only of isobutanol.

The diluent gas may be any gas that does not affect the dehydration of isobutanol, and examples thereof include nitrogen, helium, neon, krypton, xenon, radon, argon, methane, ethane, propane, butane, isobutane, carbon monoxide, carbon dioxide, nitric oxide, nitrogen dioxide, nitrous oxide, dinitrogen trioxide, dinitrogen tetroxide, dinitrogen pentoxide, and water vapor. Oxygen or hydrogen may be used as a diluent gas as long as it does not affect the dehydration of isobutanol. The diluent gas included in the raw material gas may be one kind or two or more kinds. Moisture may be included in the raw material gas.

The isobutanol concentration in the raw material gas is preferably 1.0% by volume or more, more preferably 3.0% by volume or more, still more preferably 5.0% by volume or more, particularly preferably 15.0% by volume or more, even still more preferably 25.0% by volume or more, and most preferably 45.0% by volume or more, with respect to the total volume of the raw material gas. When the isobutanol concentration is equal to or larger than the above-described lower limit value, isomerization is easily suppressed and thus the isobutylene selectivity is improved. In addition, the reactor can be easily miniaturized, the equipment cost can be reduced, and the energy cost required for the recovery of isobutylene can be reduced. The upper limit is not particularly limited, and it is 100% by volume or less.

The reaction temperature (the temperature in the catalyst layer during the reaction) in the dehydration of isobutanol is preferably 460°C or lower, more preferably 440°C or lower, still more preferably 420°C or lower, particularly preferably 400°C or lower, and most preferably 380°C or lower. When the reaction temperature is equal to or lower than the above-described upper limit value, the isomerization reaction is easily suppressed and the selectivity for isobutylene is improved. The reaction temperature in the dehydration of isobutanol is preferably 200°C or higher, more preferably 220°C or higher, still more preferably 240°C or higher, particularly preferably 260°C or higher, and most preferably 280°C or higher. When the reaction temperature is equal to or higher than the above-described lower limit value, the using amount of the catalyst and the supply amount of the raw material gas can be reduced, which is advantageous in terms of cost and productivity. The above-described upper and lower limits may be combined in any combination. For example, the reaction temperature in the dehydration of isobutanol is preferably 200°C or higher and 460°C or lower, more preferably 220°C or higher and 440°C or lower, still more preferably 240°C or higher and 420°C or lower, even still more preferably 260°C or higher and 400°C or lower, and particularly preferably 280°C or higher and 380°C or lower.

The lowest temperature, among the temperatures of the catalyst layer in the reactor, which can be confirmed after the reaction reached a steady state is defined as the reaction temperature. Therefore, when the temperature of the catalyst layer varies, it is preferable to increase the number of measurement points or measure the temperature continuously in the catalyst filling direction. The method for controlling the reaction temperature is not particularly limited, and a known method can be adopted.

The reaction pressure in the dehydration of isobutanol is not particularly limited; however, it is preferably 50 kPa or more in terms of absolute pressure. The reaction pressure is preferably 1,000 kPa or less, more preferably 900 kPa or less, still more preferably 800 kPa or less, particularly preferably 700 kPa or less, and most preferably 600 kPa or less.

The value of the reaction pressure is a value measured by a pressure sensor installed at a position where the influence of the pressure loss can be ignored with respect to the pressure at the inlet of the reactor.

[Method for producing methacrylic acid]

The method for producing methacrylic acid according to the present invention is a method of producing methacrylic acid using isobutylene produced according to the method for producing isobutylene according to the present invention. Examples thereof include the following method (A) and method (B). According to the method (A) and the method (B), methacrylic acid can be produced from isobutylene with a high selectivity.

The method (A) includes a step (a1) of producing isobutylene according to the method for producing isobutylene according to the present invention and a step (a2) of producing methacrylic acid according to the gas phase oxidation of isobutylene.

The method (B) includes a step (b1) of producing isobutylene according to the method for producing isobutylene according to the present invention, a step (b2) of hydrating isobutylene to produce tert-butyl alcohol, and a step (b3) of producing methacrylic acid according to the gas phase oxidation of the tert-butyl alcohol produced by hydrating isobutylene.

The hydration of isobutylene in the step (b2) can be carried out by a known method. Examples of the acid catalyst that is used in the hydration of isobutylene include an ion exchange resin and a heteropolyacid. The acid catalyst is preferably a strongly acidic cation exchange resin from the viewpoint that tert-butyl alcohol can be produced in a high yield.

The gas phase oxidation of isobutylene in the step (a2) and the gas phase oxidation of the tert-butyl alcohol in the step (b3) may be carried out in one stage or in two stages. Two-stage gas phase oxidation is preferable from the viewpoint of the high methacrylic acid selectivity.

When the gas phase oxidation is carried out in two stages, it is preferable to use a catalyst for the first stage oxidation in the gas phase oxidation of the first stage (the first stage oxidation). The catalyst to be used may be a known catalyst. Among the above, it is preferably a catalyst containing at least molybdenum and bismuth.

Such a catalyst is preferably a catalyst having a composition represented by Formula (1).

$$Mo_{12}Bi_{a1}Fe_{a2}M_{a3}X_{a4}Y_{a5}Z_{a6}O_{a7} \qquad (1)$$

However, in Formula (1), Mo, Bi, Fe, and O each represent molybdenum, bismuth, iron, and oxygen. M represents at least one element selected from the group consisting of cobalt and nickel. X represents at least one element selected from the group consisting of chromium, lead, manganese, calcium, magnesium, niobium, silver, barium, tin, tantalum, and zinc. Y represents at least one element selected from the group consisting of phosphorus, boron, sulfur, selenium, tellurium, cerium, tungsten, antimony, and titanium. Z represents at least one element selected from the group consisting of lithium, sodium, potassium, rubidium, cesium, and thallium, $a1$ to $a7$ represent the atomic ratio of each element, $a1$, $a2$, $a3$, $a4$, $a5$, $a6$, and $a7$ each indicate the atomic ratio of each element to the Mo12 atom, where they are $a1 = 0.01$ to 3, $a2 = 0.01$ to 5, $a3 = 1.0$ to 12, $a4 = 0$ to 8.0, $a5 = 0$ to 5.0, and $a6 = 0.001$ to 2.0, and $a7$ is the atomic ratio of oxygen, which is required to satisfy the valence of each element.

The first stage oxidation can be carried out on a fixed bed. The catalyst layer for the first stage oxidation is not particularly limited, and it may be an undiluted layer containing only the catalyst for the first stage oxidation or may be a diluted layer containing an inert carrier. The catalyst layer for the first stage oxidation may be a single layer or may be a mixed layer composed of a plurality of layers.

The concentration of isobutylene or tert-butyl alcohol in the raw material gas for the first stage oxidation is preferably 1.0% by volume or more and more preferably 3.0% by volume or more. The concentration of isobutylene or tert-butyl alcohol in the raw material gas for the first stage oxidation is preferably 20.0% by volume or less and more preferably 10.0% by volume or less. The above-described upper and lower limits may be combined in any combination. For example, the concentration of isobutylene or tert-butyl alcohol in the raw material gas for the first stage oxidation is preferably 1.0% by volume or more and 20.0% by volume or less, more preferably 3.0% by volume or more and 20.0% by volume or less, and still more preferably 3.0% by volume or more and 10.0% by volume or less.

Although it is economical to use air as the molecular oxygen source for the first stage oxidation, it is also possible to use air enriched with pure oxygen, as necessary. The molar ratio (the volume ratio) of isobutylene or tert-butyl alcohol in a reaction gas to molecular oxygen is preferably in a range of "isobutylene or tert-butyl alcohol":"molecular oxygen" = 1:0.1 to 1:5 and more preferably in a range of 0.5 to 1:3.

It is economical to use a reaction gas by diluting it with an inert gas such as nitrogen or carbon dioxide gas, water vapor, or the like, in addition to isobutylene or tert-butyl alcohol and molecular oxygen.

The reaction pressure for the first stage oxidation is preferably in a range from atmospheric pressure to 200 kPaG.

The reaction temperature for the first stage oxidation is preferably 200°C or higher and 450°C or lower, and more preferably 250°C or higher and 400°C or lower.

The contact time between isobutylene or tert-butyl alcohol and molecular oxygen in the first stage oxidation is preferably 0.5 seconds or more and more preferably 1.0 seconds or more. The contact time between isobutylene or tert-butyl alcohol and molecular oxygen in the first stage oxidation is preferably 10.0 seconds or less and more preferably 6.0 seconds or less. The above-described upper and lower limits may be combined in any combination. For example, the contact time between isobutylene or tert-butyl alcohol and molecular oxygen in the first stage oxidation is preferably 0.5 seconds or more and 10.0 seconds or less, more preferably 0.5 seconds or more and 6.0 seconds or less, and still more preferably 1.0 seconds or more and 6.0 seconds or less.

Methacrolein and methacrylic acid is obtained by the first stage oxidation. Methacrolein is converted to methacrylic acid by the gas phase oxidation of the second stage (the second stage oxidation).

A known catalyst can be used as the catalyst for the second stage oxidation, which is used in the second stage oxidation. The catalyst to be used is preferably a catalyst containing at least molybdenum and phosphorus.

Such a catalyst is preferably a catalyst having a composition represented by Formula (2).

$$P_{a8}Mo_{a9}V_{a10}Cu_{a11}A_{a12}E_{a13}Ga_{14}O_{a15} \qquad (2)$$

In Formula (2), P, Mo, V, Cu, and O each represent phosphorus, molybdenum, vanadium, copper, and oxygen. A represents at least one element selected from the group consisting of antimony, bismuth, arsenic, germanium, zirconium, tellurium, silver, selenium, silicon, tungsten, and boron. E represents at least one element selected from the group consisting of potassium, rubidium, cesium, thallium, magnesium, and barium. G represents at least one element selected from the group consisting of iron, zinc, chromium, calcium, strontium, tantalum, cobalt, nickel, manganese, titanium, tin, lead, niobium, indium, sulfur, palladium, gallium, cerium, and lanthanum. a8 to a 15 represent the atomic ratio of each element. When $a9 = 12$, they are each $a8 = 0.5$ to 3, $a10 = 0.01$ to 3, $a11 = 0.01$ to 2, $a12 = 0$ to 3, preferably 0.01 to 3, $a13 = 0.01$ to 3, $a14 = 0$ to 4, and a15 is the atomic ratio of oxygen, which is required to satisfy the valence of each element.

The second stage oxidation can be carried out on a fixed bed. The catalyst layer for the second stage oxidation is not particularly limited, and it may be an undiluted layer containing only the catalyst for the second stage oxidation or may be a diluted layer containing an inert carrier. The catalyst layer for the second stage oxidation may be a single layer or may be a mixed layer composed of a plurality of layers.

The concentration of methacrolein in the reaction gas of the second stage oxidation is not limited and can be set to any concentration; however, it is preferably 1.0% by volume or more and more preferably 3.0% by volume or more. In addition, the concentration of methacrolein in the reaction gas of the second stage oxidation is preferably 20.0% by volume or less and more preferably 10.0% by volume or less. The above-described upper and lower limits may be combined in any combination. For example, the concentration of methacrolein in the reaction gas of the second stage oxidation is preferably 1.0% by volume or more and 20.0% by volume or less, more preferably 1.0% by volume or more and 10.0% by volume or less, and still more preferably 3.0% by volume or more and 10.0% by volume or less.

Although it is economical to use air as the molecular oxygen source for the second stage oxidation, it is also possible to use air enriched with pure oxygen, as necessary.

The concentration of molecular oxygen in the reaction gas of the second stage oxidation is preferably 0.5 mol or more and more preferably 1.0 mol or more with respect to 1.0 mol of methacrolein. In addition, the molecular oxygen concentration in the reaction gas of the second stage oxidation is preferably 4.0 mol or less and more preferably 3.0 mol or less with respect to 1.0 mol of methacrolein. The above-described upper and lower limits may be combined in any combination. For example, the concentration of molecular oxygen in the second stage oxidation is preferably 0.5 mol or more and 4.0 mol or less, more preferably 1.0 mol or more and 4.0 mol or less, and still more preferably 1.0 mol or more and 3.0 mol or less with respect to 1.0 mol of methacrolein.

The reaction gas for the second stage oxidation preferably contains water (water vapor) in addition to methacrolein and molecular oxygen. In a case of carrying out the reaction in the presence of water, methacrylic acid can be obtained at a higher yield. The concentration of water vapor in the reaction gas of the second stage oxidation is preferably 0.1% by volume or more and more preferably 1.0% by volume or more. The concentration of water vapor in the reaction gas of the second stage oxidation is preferably 50.0% by volume or less and more preferably 40.0% by volume or less. The above-described upper and lower limits may be combined in any combination. For example, the concentration of water vapor in the reaction gas of the second stage oxidation is preferably 0.1% by volume or more and 50.0% by volume or less, more preferably 1.0% by volume or more and 50.0% by volume or less, and still more preferably 1.0% by volume or more and 40.0% by volume or less.

To the reaction gas for the second stage oxidation, water (water vapor) may be added in addition to methacrolein and molecular oxygen.

The reaction pressure for the second stage oxidation can be set in a range from atmospheric pressure to several hundred kPaG. The reaction temperature for the second stage oxidation is preferably 230°C or higher and more preferably 250°C or higher. In addition, the reaction temperature for the second stage oxidation is preferably 450°C or lower and more preferably 400°C or lower. The above-described upper and lower limits may be combined in any combination. For example, the reaction temperature for the second stage oxidation is preferably 230°C or higher and 400°C or lower, more preferably 250°C or higher and 450°C or lower, and still more preferably 250°C or higher and 400°C or lower.

[Method for producing methyl methacrylate]

The method for producing methyl methacrylate according to the present invention is a method of producing methyl methacrylate using methacrylic acid produced by using the method for producing methacrylic acid according to the present invention.

The method for producing methyl methacrylate according to the present invention includes a step of producing methyl methacrylate by subjecting methacrylic acid produced using the method for producing methacrylic acid according to the present invention to esterification with methanol. According to the method according to the present invention, methyl methacrylate can be produced from isobutylene with a high selectivity.

For example, methacrylic acid produced according to the production method according to the present invention is recovered by extraction, distillation operation, or the like, and it is subjected to esterification with methanol in the presence of an acid catalyst.

It is preferable to use a catalyst for esterification. The catalyst to be used is preferably an acid catalyst, and for example, sulfuric acid or an ion exchange resin can be used. The ion exchange resin is preferably a strongly acidic cation exchange resin. Examples of the strongly acidic cation exchange resin include DIAION (registered trade name), PK216, RCP12H (manufactured by Mitsubishi Chemical Corporation), LEWATIT (registered trade name), K2431 (manufactured by Bayer AG), and Amberlyst (registered trade name) 15WET (manufactured by Rohm and Haas Company, Japan). These may be used alone or in a combination of two or more thereof.

The flow direction of the reaction fluid in esterification may be either vertically upward or vertically downward, and it can be appropriately selected. When the swelling of the ion exchange resin that is used as the acid catalyst for esterification is large, the flow direction of the reaction fluid is preferably vertically upward. When the reaction fluid forms a non-uniform phase, the flow direction of the reaction fluid is preferably vertically downward.

In a case of carrying out esterification by filling the fixed bed type reactor with an ion exchange resin, the flow-through amount of the raw material including methacrylic acid and methanol is preferably 0.10 times or more and more preferably 0.20 times or more in terms of the mass ratio to the amount of the ion exchange resin. In addition, the flow-through amount of the raw material is preferably 10.0 times or less and more preferably 5.0 times or less in terms of the mass ratio with respect to the amount of the ion exchange resin. The above-described upper and lower limits may be combined in any combination. For example, the flow-through amount of the raw material including methacrylic acid and methanol is preferably 0.10 times or more and 10.0 times or less, more preferably 0.20 times or more and 10.0 times or less, and still more preferably 0.20 times or more and 5.0 times or less, in terms of the mass ratio to the amount of ion exchange resin.

When a strongly acidic cation exchange resin is used as the acid catalyst, the reaction temperature of the esterification is preferably 40°C or higher and 130°C or lower.

When the reaction temperature is 40°C or higher, the reaction rate is high, and esterification can be carried out efficiently. When the reaction temperature is 130°C or lower, the deterioration rate of the ion exchange resin is low, and the esterification can be continuously carried out for a long time. The reaction temperature for esterification can be appropriately determined to the optimum temperature from the viewpoint of chemical equilibrium.

Regarding the raw material composition, from the viewpoint of chemical equilibrium, the process of the recovery step and the purification step can be simplified by increasing the concentration of any one of methacrylic acid or methanol and increasing the conversion rate of the raw material thereof having the lower concentration.

In the present invention, as a catalyst for producing isobutylene from isobutanol, a catalyst containing a specific metal in alumina consisting of one or more crystal phases of a monoclinic crystal phase, a tetragonal crystal phase, and a cubic crystal phase is used. This promotes the dehydration of isobutanol via diisobutyl ether on alumina, and thus isobutylene can be produced with a high selectivity. As a result, methacrylic acid and methyl methacrylate can also be efficiently produced.

[Examples]

Hereinafter, the present invention will be specifically described with reference to Examples; however, the present invention is not limited to the following description.

The analysis of the raw material gas and the product was carried out using gas chromatography. The isobutanol conversion rate and the selectivity for each product are defined as follows.

$$\text{Isobutanol conversion rate (\%)} = (b/a) \times 100$$

$$\text{C4 gas selectivity (\%)} = (j/b) \times 100$$

$$\text{Diisobutyl ether selectivity (\%)} = (h/b) \times 2 \times 100$$

$$\text{Isobutyl aldehyde selectivity (\%)} = (i/b) \times 100$$

$$\text{Isobutylene selectivity in C4 gas (\%)} = (c/j) \times 100$$

$$\text{Isobutane selectivity in C4 gas (\%)} = (d/j) \times 100$$

$$\text{1-butene selectivity in C4 gas (\%)} = (e/j) \times 100$$

$$\text{trans-2-Butene selectivity in C4 gas (\%)} = (f/j) \times 100$$

$$\text{cis-2-Butene selectivity in C4 gas (\%)} = (g/j) \times 100$$

a: Number of moles of isobutanol supplied
b: Number of moles of isobutanol reacted
c: Number of moles of isobutylene generated
d: Number of moles of isobutane generated
e: Number of moles of 1-butene generated
f: Number of moles of trans-2-butene generated
g: Number of moles of cis-2-butene generated
h: Number of moles of diisobutyl ether generated
i: Number of moles of isobutyl aldehyde generated
j: Number of moles of C4 gas generated (isobutene, isobutane, 1-butene, trans-2-butene, and cis-2-butene)

The weight hourly space velocity (WHSV) per unit time of the raw material gas is defined by the following Expression (3).

$$\text{WHSV (/h)} = W1/W2 \qquad (3)$$

However, in Expression (3), W1 is the supply amount (g/h) of isobutanol per unit time. W2 is the amount (g) of the catalyst used.

[Example 1]

(Catalyst preparation method)

To a cobalt acetate aqueous solution obtained by dissolving 0.885 g of cobalt acetate (II) dihydrate (manufactured by FUJIFILM Wako Pure Chemical Corporation, purity 99.0%) in 100 mL of pure water, 50.0 g of alumina (alumina containing $\gamma$-alumina having a tetragonal crystal phase as a main component, BET specific surface area: 243 m$^2$/g, Na$_2$O content: less than 0.050% by mass, SiO$_2$ Content: 0.10% by mass) molded in a columnar pellet shape (diameter: 3.0 mm) was added, and then the solvent was removed while stirring with a rotary evaporator. After drying at 24°C for 12 hours, calcination was carried out at 600°C for 3 hours using an electric furnace to obtain a catalyst A1 having a Co content of 0.10 mmol/g with respect to 1 g of alumina.

(Reaction evaluation method)

A fixed bed reactor (a vertical-type tubular-shaped reaction tube, inner diameter: 16.3 mm, length: 500 mm) which had been immersed in a heat medium was filled with 20.1 g of the catalyst A1 to form a catalyst layer. The heat medium temperature was adjusted to 340°C. Next, isobutanol (manufactured by Nacalai Tesque, Inc., the amount of water measured according to the Karl Fischer method: 411 ppm) was adjusted to a flow rate of 0.341 ml/min using a double plunger pump and supplied to a vaporizer set at 200°C. The isobutanol concentration in the raw material gas supplied to the catalyst layer was 100% by volume.

After 1 hour had passed since the raw material gas was supplied to the reactor, the reaction evaluation was started. After the reaction reached a steady state, the gas on the outlet side of the reactor was sampled and subjected to gas chromatography (manufactured by Shimadzu Corporation, product name: GC-8A) to quantify isobutylene, isobutane, 1-butene, cis-2-butene, and trans-2-butene. In addition, the reaction gas discharged from the outlet side of the reactor was trapped in ice-cooled water, and subjected to gas chromatography (manufactured by Shimadzu Corporation, product name: GC-2014) to quantify unreacted isobutanol, diisobutyl ether, and isobutyl aldehyde. A pressure gauge for measuring the reaction pressure was installed between the vaporizer and the reactor inlet.

It is noted that it was confirmed that the pressure loss from the vaporizer to the reactor inlet is negligibly small in all the flow rate ranges under the conditions in Examples 1 to 24 and Comparative Examples 1 to 3.

[Example 2]

A catalyst B1 having an Mn content of 0.10 mmol/g with respect to 1 g of alumina was obtained in the same manner as in Example 1 except that 1.23 g of manganese acetate (II) tetrahydrate (manufactured by FUJIFILM Wako Pure Chemical Corporation, purity 99.0%) was used instead of cobalt (II) acetate dihydrate.

Isobutanol was brought into contact with the catalyst B1 in the same manner as in Example 1 to obtain a product except that 20.1 g of the catalyst B1 was used instead of the catalyst A1 and the isobutanol supply rate was changed to 0.308 ml/min.

[Example 3]

A catalyst C1 having a Ni content of 0.10 mmol/g with respect to 1 g of alumina was obtained in the same manner as in Example 1 except that 1.24 g of nickel acetate (II) tetrahydrate (manufactured by FUJIFILM Wako Pure Chemical Corporation, purity 98.0%) was used instead of cobalt (II) acetate dihydrate.

Isobutanol was brought into contact with the catalyst C1 in the same manner as in Example 1 to obtain a product except that 20.0 g of the catalyst C1 was used instead of the catalyst A1 and the isobutanol supply rate was changed to 0.327 ml/min.

[Example 4]

A catalyst D1 having a Zn content of 0.10 mmol/g with respect to 1 g of alumina was obtained in the same manner as in Example 1 except that 1.10 g of zinc acetate dihydrate (manufactured by FUJIFILM Wako Pure Chemical Corporation, purity 98.0%) was used instead of cobalt (II) acetate dihydrate.

Isobutanol was brought into contact with the catalyst D1 in the same manner as in Example 1 to obtain a product except that 20.0 g of the catalyst D1 was used instead of the catalyst A1 and the isobutanol supply rate was changed to 0.266 ml/min.

[Comparative Example 1]

Isobutanol was brought into contact with the catalyst E1 in the same manner as in Example 1 to obtain a product except that alumina obtained by calcination of alumina (alumina containing γ-alumina having a tetragonal crystal phase as a main component, BET specific surface area: 243 $m^2$/g, Na$_2$O content: less than 0.050% by mass, SiO$_2$ Content: 0.10% by mass) molded in a columnar pellet shape (diameter: 3.0 mm) at 600°C for 3 hours was used as 14.4 g of the catalyst E1 and the isobutanol supply rate was changed to 2.57 ml/min.

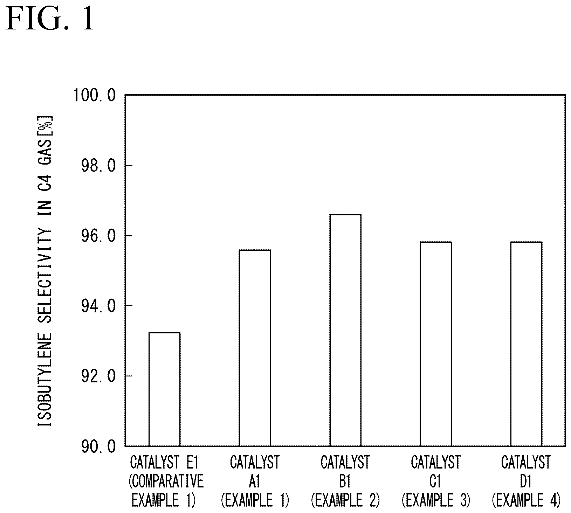

Table 1 shows the reaction conditions and evaluation results in Examples 1 to 4 and Comparative Example 1. Fig. 1 shows the measurement results of the isobutanol conversion rate, the C4 gas selectivity in the product, and the isobutylene selectivity in the C4 gas.

[Table 1]

| | | Example 1 | Example 2 | Example 3 | Example 4 | Comparative Example 1 |
|---|---|---|---|---|---|---|
| Supply amount of raw material | Isobutanol [ml/minute] | 0.341 | 0.308 | 0.327 | 0.266 | 2.570 |
| Composition of raw material gas | Isobutanol [% by volume] | 100 | 100 | 100 | 100 | 100 |
| Catalyst | Kind | A1 | B1 | C1 | D1 | E1 |
| | Supported metal | Co | Mn | Ni | Zn | - |
| | Amount of metal supported [mmol/g-alumina] | 0.10 | 0.10 | 0.10 | 0.10 | 0 |
| Catalyst amount [g] | | 20.1 | 20.1 | 20.0 | 20.0 | 14.4 |
| WHSV [/h] | | 0.82 | 0.82 | 0.82 | 0.82 | 1.14 |
| Conversion rate [%] | | 92.6 | 55.3 | 93.5 | 92.1 | 95.1 |
| Selectivity [%] | C4 gas | 99.4 | 97.6 | 84.1 | 98.0 | 97.3 |
| | Diisobutyl ether | 0.4 | 1.5 | 13.7 | 1.1 | 1.4 |
| | Isobutyl aldehyde | 0.2 | 0.9 | 2.2 | 0.9 | 1.3 |
| Selectivity in C4 gas [%] | Isobutylene | 95.5 | 96.2 | 95.6 | 95.7 | 93.1 |
| | Isobutane | 0.2 | 0.4 | 0.1 | 0.1 | 0.1 |
| | 1-butene | 1.9 | 1.6 | 1.7 | 1.7 | 2.6 |
| | cis-2-butene | 1.8 | 1.3 | 1.9 | 1.8 | 3.0 |
| | trans-2-butene | 0.6 | 0.5 | 0.7 | 0.7 | 1.2 |

As shown in Table 1 and Fig. 1, in Examples 1 to 4 in which the catalysts A1 to D1 obtained by incorporating Co, Mn, Ni, and Zn into alumina are used, the isobutylene selectivity is high as compared with Comparative Example 1 in which the catalyst E1 containing no metal is used.

[Examples 5 to 12]

Catalysts D2 to D9 were obtained in the same manner as in Example 1 except that zinc acetate dihydrate was used instead of cobalt (II) acetate dihydrate and the Zn content was changed as shown in Table 2.

Isobutanol was brought into contact with the catalysts D2 to D9 in the same manner as in Example 1 to obtain a product except that the catalysts D2 to D9 were used instead of the catalyst A1, and the amount of the catalyst with which the reaction tube is filled, the isobutanol supply amount to the reaction tube, and the WHSV were adjusted as shown in Table 2.

[Comparative Example 2]

Isobutanol was brought into contact with the catalyst E1 in the same manner as in Example 1 to obtain a product except that the catalysts E1 was used, and the amount of the catalyst with which the reaction tube is filled, the isobutanol supply amount to the reaction tube and the WHSV were adjusted as shown in Table 2.

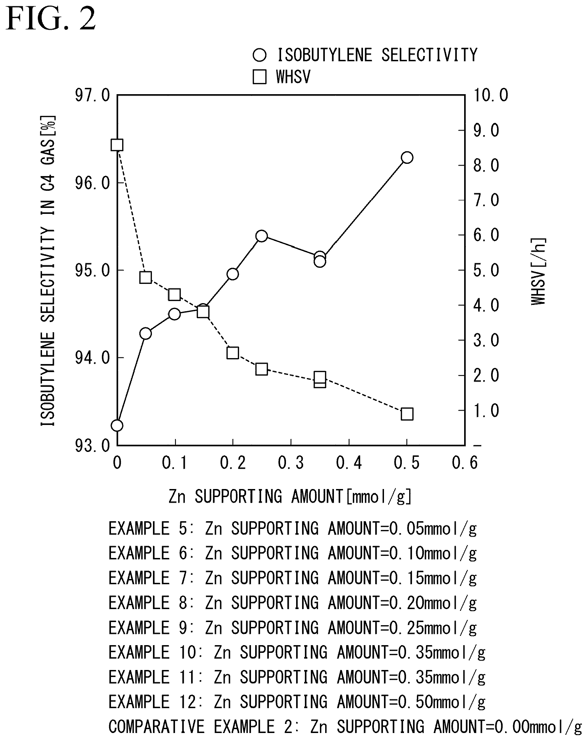

Table 2 shows the reaction conditions and evaluation results in Examples 5 to 12 and Comparative Example 2. In addition, Fig. 2 shows a graph showing the isobutylene selectivity in the C4 gas and WHSV of each example.

[Table 2]

| | | Example 5 | Example 6 | Example 7 | Example 8 | Example 9 | Example 10 | Example 11 | Example 12 | Comparative Example 2 |
|---|---|---|---|---|---|---|---|---|---|---|
| Supply amount of raw material | Isobutanol [ml/minute] | 0.922 | 0.834 | 0.738 | 0.509 | 0.426 | 0.357 | 0.382 | 0.176 | 1.65 |
| Composition of raw material gas | Isobutanol [% by volume] | 100 | 100 | 100 | 100 | 100 | 100 | 100 | 100 | 100 |
| Catalyst | Kind | D2 | D3 | D4 | D5 | D6 | D7 | D8 | D9 | E1 |
| | Supported metal | Zn | Zn | Zn | Zn | Zn | Zn | Zn | Zn | - |
| | Amount of zinc acetate dihydrate used in supporting [g] | 0.549 | 1.10 | 1.65 | 2.20 | 2.74 | 3.84 | 3.84 | 5.49 | 0.00 |
| | Amount of Zn supported [mmol/g-alumina] | 0.05 | 0.10 | 0.15 | 0.20 | 0.25 | 0.35 | 0.35 | 0.50 | 0.00 |
| Catalyst amount [g] | | 14.4 | 14.5 | 14.5 | 14.4 | 14.5 | 14.4 | 14.4 | 14.5 | 14.4 |
| Supply amount of isobutanol [g/h] | | 69.0 | 62.4 | 55.2 | 38.1 | 31.9 | 26.7 | 28.6 | 13.2 | 123.8 |
| WHSV [/h] | | 4.8 | 4.3 | 3.8 | 2.6 | 2.2 | 1.9 | 2.0 | 0.9 | 8.6 |
| Conversion rate [%] | | 94.5 | 93.7 | 93.3 | 95.6 | 95.3 | 93.5 | 93.1 | 91.3 | 95.1 |
| Selectivity [%] | C4 gas | 99.4 | 99.2 | 99.2 | 99.3 | 99.0 | 98.5 | 98.4 | 96.3 | 99.6 |
| | Diisobutyl ether | 0.2 | 0.3 | 0.4 | 0.3 | 0.4 | 0.7 | 0.8 | 2.0 | 0.2 |
| | Isobutyl aldehyde | 0.4 | 0.5 | 0.4 | 0.4 | 0.6 | 0.8 | 0.8 | 1.7 | 0.2 |
| Selectivity in C4 gas [%] | Isobutylene | 94.2 | 94.4 | 94.5 | 94.9 | 95.2 | 94.4 | 95.0 | 94.9 | 93.1 |
| | Isobutane | 0.1 | 0.1 | 0.1 | 0.1 | 0.1 | 0.1 | 0.1 | 0.1 | 0.1 |
| | 1-butene | 2.2 | 2.1 | 2.1 | 2.1 | 1.9 | 1.9 | 2.2 | 2.0 | 2.6 |
| | cis-2-butenc | 2.7 | 2.6 | 2.5 | 2.1 | 2.1 | 2.9 | 2.0 | 2.2 | 3.0 |
| | trans-2-butene | 0.8 | 0.8 | 0.8 | 0.8 | 0.7 | 0.7 | 0.7 | 0.8 | 1.2 |

As shown in Table 2 and Fig. 2, in Examples 5 to 12 in which the catalysts D2 to D9 obtained by incorporating Zn into alumina are used, the isobutylene selectivity is high as compared with Comparative Example 2 in which the catalyst E1 containing no metal is used. In addition, the higher the Zn content is, the higher the isobutylene selectivity tends to be.

[Examples 13 to 17]

Catalysts D10 to D14 were obtained in the same manner as in Example 1 except that zinc acetate was used instead of cobalt (II) acetate dihydrate and the calcination temperature was set to 700°C, and the Zn content was changed as shown in Table 3.
Isobutanol was brought into contact with the catalysts D10 to D14 in the same manner as in Example 1 to obtain a product except that the catalysts D10 to D14 were used instead of the catalyst A1, and the amount of the catalyst with which the reaction tube is filled, the isobutanol supply amount to the reaction tube, and the WHSV were adjusted as shown in Table 3.

[Comparative Example 3]

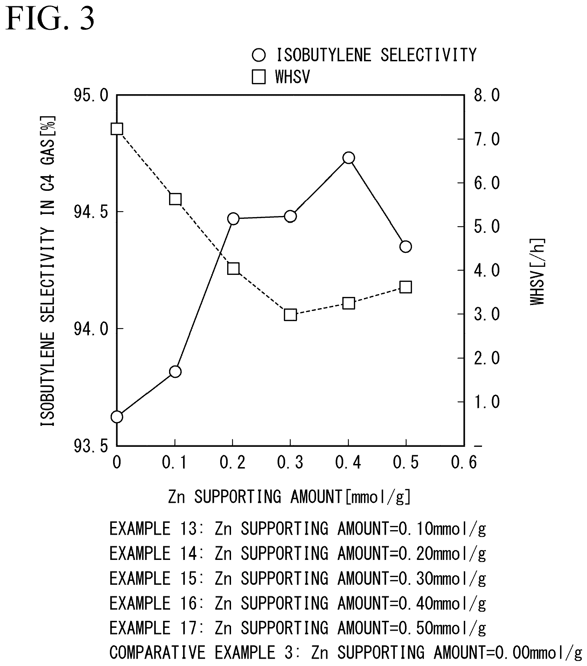

Alumina (alumina containing $\gamma$-alumina having a tetragonal crystal phase as a main component, BET specific surface area: 243 $m^2$/g, $Na_2O$ content: less than 0.050% by mass, $SiO_2$ Content: 0.10% by mass) molded in a columnar pellet shape (diameter: 3.0 mm) calcined at 700°C for 3 hours was used as a catalyst E2.
Isobutanol was brought into contact with the catalyst E2 in the same manner as in Example 1 to obtain a product except that the catalyst E2 was used instead of the catalyst A1, and the amount of the catalyst with which the reaction tube is filled, the isobutanol supply amount to the reaction tube, and the WHSV were adjusted as shown in Table 3.
Table 3 shows the reaction conditions and evaluation results in Examples 13 to 17 and Comparative Example 3. In addition, Fig. 3 shows a graph showing the isobutylene selectivity in the C4 gas and WHSV of each example.

[Table 3]

| | | Example 13 | Example 14 | Example 15 | Example 16 | Example 17 | Comparative Example 3 |
|---|---|---|---|---|---|---|---|
| Supply amount of raw material | Isobutanol [ml/minute] | 1.09 | 0.777 | 0.572 | 0.630 | 0.700 | 1.40 |
| Composition of raw material gas | Isobutanol [% by volume] | 100 | 100 | 100 | 100 | 100 | 100 |
| Catalyst | Kind | D10 | D11 | D12 | D13 | D14 | E2 |
| | Supported metal | Zn | Zn | Zn | Zn | Zn | - |
| | Amount of zinc acetate dihydrate used in supporting [g] | 1.10 | 2.20 | 3.30 | 4.40 | 5.50 | 0.00 |
| | Amount of Zn supported [mmol/g-alumina] | 0.10 | 0.20 | 0.30 | 0.40 | 0.50 | 0 |
| Catalyst amount [g] | | 14.5 | 14.4 | 14.4 | 14.5 | 14.5 | 14.5 |
| Supply amount of isobutanol [g/h] | | 81.4 | 58.1 | 42.8 | 47.1 | 52.4 | 104.7 |
| WHSV [/h] | | 5.6 | 4.0 | 3.0 | 3.3 | 3.6 | 7.2 |
| Conversion rate [%] | | 97.1 | 93.3 | 96.2 | 96.6 | 96.0 | 96.4 |
| Selectivity [%] | C4 gas | 99.8 | 99.6 | 99.7 | 99.7 | 99.7 | 99.8 |
| | Diisobutyl ether | 0.1 | 0.3 | 0.2 | 0.2 | 0.2 | 0.1 |
| | Isobutyl aldehyde | 0.1 | 0.1 | 0.1 | 0.1 | 0.1 | 0.1 |

(continued)

| | | Example 13 | Example 14 | Example 15 | Example 16 | Example 17 | Comparative Example 3 |
|---|---|---|---|---|---|---|---|
| Selectivity in C4 gas [%] | Isobutylene | 93.7 | 94.4 | 94.4 | 94.6 | 94.2 | 93.5 |
| | Isobutane | 0.1 | 0.1 | 0.1 | 0.1 | 0.1 | 0.1 |
| | 1-butene | 2.4 | 2.3 | 2.3 | 2.2 | 2.4 | 2.5 |
| | cis-2-butene | 2.8 | 2.3 | 2.3 | 2.2 | 2.4 | 2.8 |
| | trans-2-butene | 1.0 | 0.9 | 0.9 | 0.9 | 0.9 | 1.1 |

As shown in Table 3 and Fig. 3, in Examples 13 to 17 in which the catalysts D10 to D14 obtained by incorporating Zn into alumina are used, the isobutylene selectivity is high as compared with Comparative Example 3 in which the catalyst E2 containing no metal is used. In addition, the higher the Zn content is, the higher the isobutylene selectivity tends to be, even when the calcination temperature of the catalyst is 700°C.

[Examples 18 to 24]

Catalysts D15 to D21 were obtained in the same manner as in Example 1 except that zinc acetate was used instead of cobalt acetate and the calcination temperature was changed as shown in Table 4.

Isobutanol was brought into contact with the catalysts D15 to D21 in the same manner as in Example 1 to obtain a product except that the catalysts D15 to D21 were used instead of the catalyst A1, and the amount of the catalyst with which the reaction tube is filled, the isobutanol supply amount to the reaction tube, and the WHSV were adjusted as shown in Table 4.

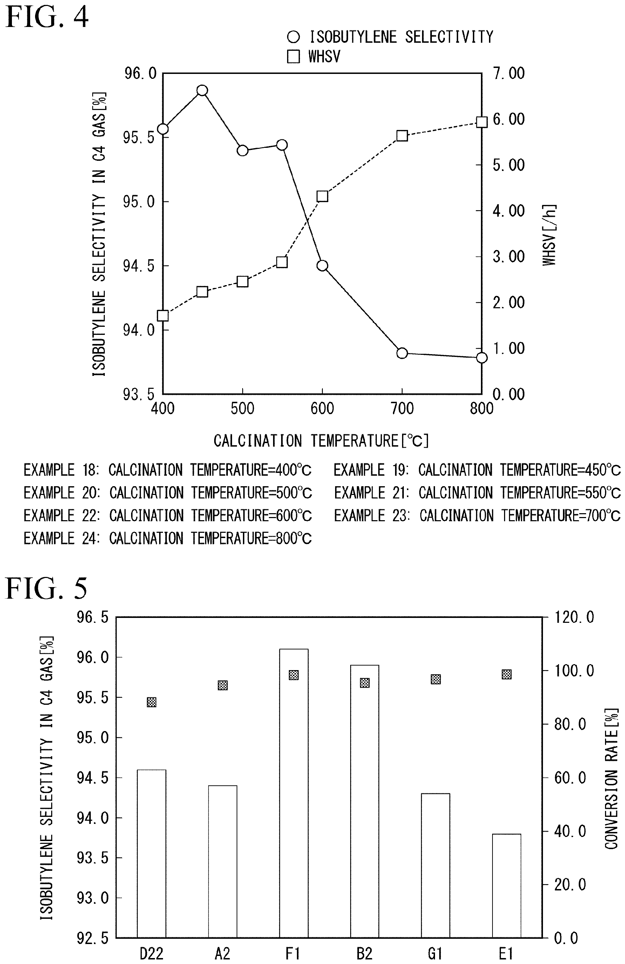

Table 4 shows the reaction conditions and evaluation results in Examples 18 to 24. In addition, Fig. 4 shows a graph showing the isobutylene selectivity in the C4 gas and WHSV of each example.

[Table 4]

| | | Example 18 | Example 19 | Example 20 | Example 21 | Example 22 | Example 23 | Example 24 |
|---|---|---|---|---|---|---|---|---|
| Supply amount of raw material | Isobutanol [ml/minute] | 0.331 | 0.430 | 0.478 | 0.560 | 0.834 | 1.09 | 1.14 |
| Composition of raw material gas | Isobutanol [% by volume] | 100 | 100 | 100 | 100 | 100 | 100 | 100 |
| Catalyst | Kind | D15 | D16 | 1)17 | D18 | 1)19 | D20 | 1)21 |
| | Supported metal | Zn | in | Zn | Zn | Zn | Zn | Zn |
| | Amount of Zn supported [mmol/g-alumina] | 0.10 | 0.10 | 0.10 | 0.10 | 0.10 | 0.10 | 0.10 |
| | Calcination temperature [°C] | 400 | 450 | 500 | 550 | 600 | 700 | 800 |
| Catalyst amount [g] | | 14.5 | 14.4 | 14.5 | 14.5 | 14.5 | 14.5 | 14.4 |
| Supply amount of isobutanol [g/h] | | 24.8 | 32.0 | 35.7 | 41.9 | 62.4 | 81.4 | 85.2 |
| WHSV [/h] | | 1.71 | 2.22 | 2.47 | 2.89 | 4.32 | 5.62 | 5.92 |
| Conversion rate [%] | | 94.3 | 93.3 | 94.3 | 95.3 | 93.7 | 97.1 | 94.0 |
| Selectivity [%] | C4 gas | 98.4 | 99.0 | 99.1 | 99.2 | 99.2 | 99.7 | 99.5 |
| | Diisobutyl ether | 0.4 | 0.3 | 0.3 | 0.3 | 0.3 | 0.1 | 0.3 |
| | Isobutyl aldehyde | 1.2 | 0.7 | 0.6 | 0.5 | 0.5 | 0.2 | 0.2 |
| Selectivity in C4 gas [%] | Isobutylene | 95.5 | 95.8 | 95.3 | 95.3 | 94.4 | 93.7 | 93.7 |
| | Isobutane | 0.1 | 0.1 | 0.1 | 0.1 | 0.1 | 0.1 | 0.1 |
| | 1 - butene | 1.7 | 1.7 | 1.8 | 1.9 | 2.1 | 2.4 | 2.4 |
| | cis-2-butene | 2.0 | 1.7 | 2.1 | 1.9 | 2.6 | 2.8 | 2.9 |
| | trans-2-butene | 0.7 | 0.7 | 0.7 | 0.8 | 0.8 | 1.0 | 0.9 |

EP 4 129 963 A1

16

As shown in Table 4 and Fig. 4, in Examples 18 to 24 in which the catalysts D15 to D21 are used, the isobutylene selectivity tends to be higher when the calcination temperature is lower in a range of 400 to 800°C.

[Example 25]

(Catalyst preparation method)

A catalyst was prepared in the same manner as in Example 4, thereby obtaining a catalyst D22 having a Zn content of 0.10 mmol with respect to 1 g of alumina, except that alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) was used.

(Reaction evaluation method)

A vertical-type tubular-shaped reaction tube having an inner diameter of 0.50 cm and a length of 40 cm was filled with 1.01 g of the catalyst D22 (crushed and sized so that the particle diameter was 800 to 1,190 $\mu$m) to form a catalyst layer. For the reactor, the set temperature of the electric furnace for the reaction tube was adjusted so that the catalyst layer temperature became a predetermined temperature. Next, isobutanol (manufactured by Nacalai Tesque, Inc., the amount of water measured according to the Karl Fischer method: 411 ppm) was introduced into a vaporizer heated at 200°C at 0.18 ml/min with a syringe pump and then vaporized. Nitrogen gas as a diluent gas was supplied to the vaporizer at a flow rate of 11 ml (standard state)/min using a mass flow meter, where it was supplied to the reactor together with the vaporized isobutanol. The isobutanol concentration in the raw material gas supplied to the catalyst layer was 79.9% by volume, and the temperature (the reaction temperature) of the catalyst layer during the reaction was 340°C.

After 1 hour had passed since the raw material gas was supplied to the reactor, the reaction evaluation was started. After the reaction reached a steady state, the gas on the outlet side of the reactor was sampled and subjected to gas chromatography (manufactured by Shimadzu Corporation, product name: GC-8A) to quantify isobutylene, isobutane, 1-butene, cis-2-butene, and trans-2-butene. In addition, the reaction gas discharged from the outlet side of the reactor was trapped in ice-cooled acetonitrile, and subjected to gas chromatography (manufactured by Shimadzu Corporation, product name: GC-2010) to quantify unreacted isobutanol, diisobutyl ether, and isobutyl aldehyde.

[Example 26]

A catalyst was prepared in the same manner as in Example 1, thereby obtaining a catalyst A2 having a Co content of 0.10 mmol with respect to 1 g of alumina, except that alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) was used.
Isobutanol was brought into contact with the catalyst A2 in the same manner as in Example 25 to obtain a product except that 1.00 g of the catalyst A2 was used instead of the catalyst D22.

[Example 27]

A catalyst was prepared in the same manner as in Example 25, thereby obtaining a catalyst F1 having a Cu content of 0.10 mmol with respect to 1 g of alumina except that alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) and 1.18 g of copper (II) nitrate dihydrate (manufactured by FUJIFILM Wako Pure Chemical Corporation, purity: 99.0% to 104.0%) were used.
Isobutanol was brought into contact with the catalyst F1 in the same manner as in Example 25 to obtain a product except that 1.52 g of the catalyst F1 was used instead of the catalyst D22.

[Example 28]

A catalyst was prepared in the same manner as in Example 2, thereby obtaining a catalyst B2 having an Mn content of 0.10 mmol with respect to 1 g of alumina, except that alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) was used.
Isobutanol was brought into contact with the catalyst B2 in the same manner as in Example 25 to obtain a product except that 2.97 g of the catalyst B2 was used instead of the catalyst D22 in a vertical-type tubular-shaped reaction tube having an inner diameter of 0.75 cm and a length of 40 cm.

[Example 29]

A catalyst was prepared in the same manner as in Example 25, thereby obtaining a catalyst G1 having a Fe content of 0.10 mmol/g with respect to 1 g of alumina except that alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) and 1.46 g of iron (III) nitrate nonahydrate (manufactured by FUJIFILM Wako Pure Chemical Corporation, purity: 99.0%) were used.

Isobutanol was brought into contact with the catalyst G1 in the same manner as in Example 25 to obtain a product except that 1.51 g of the catalyst G1 was used instead of the catalyst D22.

[Comparative Example 4]

Alumina (alumina consisting of crystal phases of $\gamma$-, $\theta$-, $\alpha$-alumina phases, particle diameter: 800 to 1,190 $\mu$m, BET specific surface area: 105 m$^2$/g, Na$_2$O content: less than 0.0500% by mass, SiO$_2$ content: 0.160% by mass) molded in a columnar pellet shape (diameter: 3.00 mm) was crushed and sized so that the particle diameter was 800 to 1,190 $\mu$m, thereby obtaining E2.

Isobutanol was brought into contact with the catalyst E2 in the same manner as in Example 25 to obtain a product except that 1.01 g of the catalyst E2 was used instead of the catalyst D22.

Table 5 shows the reaction conditions and evaluation results in Examples 25 to 29 and Comparative Example 4. In addition, Fig. 5 shows a graph showing the isobutylene selectivity and the pass-through rate of isobutanol in the C4 gas of each example.

[Table 5]

| | | Example 25 | Example 26 | Example 27 | Example 28 | Example 29 | Comparative Example 4 |
|---|---|---|---|---|---|---|---|
| Supply amount of raw material | Isobutanol [ml/minute] | 0.18 | 0.18 | 0.18 | 0.18 | 0.32 | 0.18 |
| | Nitrogen [ml (standard state)/minute] | 11 | 11 | 11 | 11 | 19 | 11.00 |
| Composition of raw material gas | Isobutanol [% by volume] | 79.9 | 79.9 | 79.9 | 79.9 | 80.1 | 79.9 |
| Catalyst | Kind | D22 | A2 | F1 | B2 | G1 | E1 |
| | Supported metal | Zn | Co | Cu | Mn | Fe | - |
| | Amount of metal supported [mmol/g-alumina] | 0.10 | 0.10 | 0.10 | 0.10 | 0.10 | - |
| | Calcination temperature [°C] | 600 | 600 | 600 | 600 | 600 | - |
| Catalyst amount [g] | | 1.01 | 1.00 | 1.52 | 2.97 | 1.51 | 1.0 1 |
| Supply amount of isobutanol [g/h] | | 8.66 | 8.66 | 8.66 | 8.66 | 15.16 | 8.66 |
| WHSV [/h] | | 8.61 | 8.62 | 5.68 | 2.92 | 10.07 | 8.62 |
| Conversion rate [%] | | 88.2 | 94.5 | 98.4 | 95.4 | 96.8 | 98.6 |
| Selectivity [%] | C4 gas | 97.7 | 99.0 | 94.6 | 99.6 | 99.8 | 99.6 |
| | Diisobutyl ether | 1.3 | 0.6 | 5.4 | 0.4 | 0.2 | 0.2 |
| | Isobutyl aldehyde | 1.0 | 0.4 | 0.0 | 0.0 | 0.0 | 0.2 |

(continued)

|  | | Example 25 | Example 26 | Example 27 | Example 28 | Example 29 | Comparative Example 4 |
|---|---|---|---|---|---|---|---|
| Selectivity in C4 gas [%] | Isobutylene | 94.6 | 94.4 | 96.1 | 95.9 | 94.3 | 93.8 |
| | Isobutanc | 0.1 | 0.1 | 0.0 | 0.1 | 0.1 | 0.1 |
| | 1-butene | 2.4 | 2.5 | 1.7 | 1.9 | 2.4 | 2.7 |
| | cis-2-butene | 2.2 | 2.2 | 1.6 | 1.6 | 2.5 | 2.5 |
| | trans-2-butene | 0.7 | 0.8 | 0.6 | 0.5 | 0.7 | 0.9 |

As shown in Table 5 and Fig. 5, in Examples 25 to 29 in which a catalyst obtained by incorporating Zn, Co, Cu, Mn, and Fe into alumina is used, the isobutylene selectivity is high as compared with Comparative Example 1 in which the catalyst E2 containing no metal is used.

**Claims**

1. A catalyst comprising:
   at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase.

2. The catalyst according to Claim 1,
   wherein a content of the metal is 0.025 mmol or more with respect to 1 g of the alumina.

3. A method for producing isobutylene, comprising:
   producing isobutylene from isobutanol using the catalyst according to Claim 1 or 2.

4. A method for producing methacrylic acid, comprising:
   producing methacrylic acid from the isobutylene produced according to the method for producing isobutylene according to Claim 3.

5. A method for producing methacrylic acid, comprising:
   obtaining tert-butyl alcohol from the isobutylene produced according to the method for producing isobutylene according to Claim 3, and subsequently producing methacrylic acid from the obtained tert-butyl alcohol.

6. A method for producing methyl methacrylate, comprising:
   producing methyl methacrylate from the methacrylic acid produced according to the method for producing methacrylic acid according to Claim 4 or 5 and from methanol.

7. A method for producing isobutylene, comprising:
   producing isobutylene from isobutanol by using a catalyst containing at least one metal selected from Group 6 to Group 14 metal elements in Period 4 to Period 6 of the periodic table, in alumina which includes alumina consisting of one or more crystal phases of a monoclinic crystal phase, a tetragonal crystal phase, and a cubic crystal phase.

8. A method for producing methacrylic acid, comprising:
   producing methacrylic acid from the isobutylene produced according to the method for producing isobutylene according to Claim 7.

9. A method for producing methacrylic acid, comprising:
   obtaining tert-butyl alcohol from the isobutylene produced according to the method for producing isobutylene according to Claim 7, and subsequently producing methacrylic acid from the obtained tert-butyl alcohol.

10. A method for producing methyl methacrylate, comprising:
    producing methyl methacrylate from the methacrylic acid produced according to the method for producing methacrylic acid according to Claim 8 or 9 and from methanol.

FIG. 1

# FIG. 2

○ ISOBUTYLENE SELECTIVITY
□ WHSV

EXAMPLE 5: Zn SUPPORTING AMOUNT=0.05mmol/g
EXAMPLE 6: Zn SUPPORTING AMOUNT=0.10mmol/g
EXAMPLE 7: Zn SUPPORTING AMOUNT=0.15mmol/g
EXAMPLE 8: Zn SUPPORTING AMOUNT=0.20mmol/g
EXAMPLE 9: Zn SUPPORTING AMOUNT=0.25mmol/g
EXAMPLE 10: Zn SUPPORTING AMOUNT=0.35mmol/g
EXAMPLE 11: Zn SUPPORTING AMOUNT=0.35mmol/g
EXAMPLE 12: Zn SUPPORTING AMOUNT=0.50mmol/g
COMPARATIVE EXAMPLE 2: Zn SUPPORTING AMOUNT=0.00mmol/g

# FIG. 3

○ ISOBUTYLENE SELECTIVITY
□ WHSV

EXAMPLE 13: Zn SUPPORTING AMOUNT=0.10mmol/g
EXAMPLE 14: Zn SUPPORTING AMOUNT=0.20mmol/g
EXAMPLE 15: Zn SUPPORTING AMOUNT=0.30mmol/g
EXAMPLE 16: Zn SUPPORTING AMOUNT=0.40mmol/g
EXAMPLE 17: Zn SUPPORTING AMOUNT=0.50mmol/g
COMPARATIVE EXAMPLE 3: Zn SUPPORTING AMOUNT=0.00mmol/g

## FIG. 4

○ ISOBUTYLENE SELECTIVITY
□ WHSV

EXAMPLE 18: CALCINATION TEMPERATURE=400°C  EXAMPLE 19: CALCINATION TEMPERATURE=450°C
EXAMPLE 20: CALCINATION TEMPERATURE=500°C  EXAMPLE 21: CALCINATION TEMPERATURE=550°C
EXAMPLE 22: CALCINATION TEMPERATURE=600°C  EXAMPLE 23: CALCINATION TEMPERATURE=700°C
EXAMPLE 24: CALCINATION TEMPERATURE=800°C

## FIG. 5

## INTERNATIONAL SEARCH REPORT

| International application No. |
| --- |
| PCT/JP2021/012965 |

**A. CLASSIFICATION OF SUBJECT MATTER**
C07C 1/24(2006.01)i; B01J 23/06(2006.01)i; B01J 23/34(2006.01)i; B01J
23/75(2006.01)i; B01J 23/755(2006.01)i; C07C 11/09(2006.01)i; C07C
51/235(2006.01)i; C07C 51/25(2006.01)i; C07C 57/05(2006.01)i; C07C
67/08(2006.01)i; C07C 69/54(2006.01)i; C07B 61/00(2006. 01)n
FI:     C07C1/24; C07C11/09; B01J23/75 Z; B01J23/34 Z; B01J23/755 Z;
        B01J23/06 Z; C07C51/25; C07C57/05; C07C67/08; C07C69/54 Z;
        C07C51/235; C07B61/00 300
According to International Patent Classification (IPC) or to both national classification and IPC

**B. FIELDS SEARCHED**

Minimum documentation searched (classification system followed by classification symbols)
C07C1/24; B01J23/00-23/96; C07C11/09; C07C51/235; C07C51/25; C07C57/05;
C07C67/08; C07C69/54; C07B61/00

Documentation searched other than minimum documentation to the extent that such documents are included in the fields searched
Published examined utility model applications of Japan          1922-1996
Published unexamined utility model applications of Japan        1971-2021
Registered utility model specifications of Japan                1996-2021
Published registered utility model applications of Japan        1994-2021

Electronic data base consulted during the international search (name of data base and, where practicable, search terms used)
CAplus/REGISTRY (STN); CASREACT (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | US 2012/0053384 A1 (LEE, Ivan C.) 01 March 2012 (2012-03-01) claims 1-20, paragraph [0032], page 5, tables | 1-3, 7<br>4-6, 8-10 |
| Y<br>A | WO 2016/002649 A1 (MITSUBISHI RAYON CO., LTD.) 07 January 2016 (2016-01-07) claims 1-9 | 4-6, 8-10<br>1-3, 7 |
| X | JP 53-2426 A (SOC FR PROD CATALYSE (PROCATALYSE)) ) 11 January 1978 (1978-01-11) claim (1), page 4, lower left column, lines 2-3, page 5, upper right column, line 16 to lower left column, line 17, table 1 | 1-2 |

☒ Further documents are listed in the continuation of Box C.   ☒ See patent family annex.

| | |
| --- | --- |
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 21 May 2021 (21.05.2021) | 01 June 2021 (01.06.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2021/012965

C (Continuation). DOCUMENTS CONSIDERED TO BE RELEVANT

| Category* | Citation of document, with indication, where appropriate, of the relevant passages | Relevant to claim No. |
|---|---|---|
| X | JP 2014-129331 A (TOYOTA MOTOR CORP.) 10 July 2014 (2014-07-10) claims 1, 2, paragraphs [0050]-[0060], table 1 | 1-2 |
| X | JP 8-10578 A (SANGYO SOUZOU KENKYUSHO) 16 January 1996 (1996-01-16) claims 1, 2, paragraphs [0038]-[0040], table 1 | 1-2 |
| X | JP 11-137964 A (NEC CORP.) 25 May 1999 (1999-05-25) claims 7-9, paragraph [0019] | 1-2 |
| X | JP 2012-532893 A (COMMONWEALTH SCIENTIFIC AND INDUSTRIAL RESEARCH ORGANISATION) 20 December 2012 (2012-12-20) claims 1, 5, paragraphs [0070]-[0072], table 2 | 1-2 |
| X | JP 2001-501961 A (SHELL INTERNATIONALE RESEARCH MAATSCHAPPIJ B.V.) 13 February 2001 (2001-02-13) claims 1, 7-9, example 1 | 1-2 |
| X | JP 2019-104004 A (IFP ENERGIES NOUVELLES) 27 June 2019 (2019-06-27) claims 1, 8, paragraphs [0144]-[0145] | 1-2 |
| X<br>A | CN 105413670 A (TSINGHUA UNIVERSITY) 23 March 2016 (2016-03-23) claims 1-10 | 1-2<br>3, 7 |
| A | WO 2014/046118 A1 (NATIONAL UNIVERSITY CORPORATION HOKKAIDO UNIVERSITY) 27 March 2014 (2014-03-27) entire text | 1-10 |
| A | CN 106582603 A (CHINA PETROLEUM & CHEMICAL CORPORATION) 26 April 2017 (2017-04-26) entire text | 1-10 |
| A | CN 107118069 A (ANHUI HAIDE PETROCHEMICAL CO., LTD.) 01 September 2017 (2017-09-01) entire text | 1-10 |
| A | JP 2013-522271 A (TOTAL RESEARCH & TECHNOLOGY FELUY) 13 June 2013 (2013-06-13) entire text | 1-10 |
| A | JP 2017-537781 A (CHINA PETROLEUM & CHEMICAL CORPORATION) 21 December 2017 (2017-12-21) entire text | 1-10 |

Form PCT/ISA/210 (continuation of second sheet) (January 2015)

**INTERNATIONAL SEARCH REPORT**
Information on patent family members

International application No.

PCT/JP2021/012965

| Patent Documents referred in the Report | Publication Date | Patent Family | Publication Date |
|---|---|---|---|
| US 2012/0053384 A1 | 01 Mar. 2012 | (Family: none) | |
| WO 2016/002649 A1 | 07 Jan. 2016 | US 2017/0129844 A1 claims 1-12 KR 10-2016-0148677 A CN 106470963 A | |
| JP 53-2426 A | 11 Jan. 1978 | US 4138443 A claim 1, column 5, lines 44-68, table 1 DE 2727759 A FR 2355791 A | |
| JP 2014-129331 A | 10 Jul. 2014 | WO 2014/083426 A1 claims 1-2, paragraphs [0051]-[0061], fig. 3 | |
| JP 8-10578 A | 16 Jan. 1996 | (Family: none) | |
| JP 11-137964 A | 25 May 1999 | US 2001/0048890 A1 claims 7-9, paragraph [0025] | |
| JP 2012-532893 A | 20 Dec. 2012 | US 2012/0264988 A1 claims 1, 5, paragraphs [0065]-[0067], table 2 EP 2805934 A1 CN 102482178 A KR 10-2012-0073197 A | |
| JP 2001-501961 A | 13 Feb. 2012 | WO 1998/016494 A1 claims 1, 7-9, example 1 KR 10-2000-0049083 A CN 1232445 A | |
| JP 2019-104004 A | 27 Jun. 2019 | US 2019/0185764 A1 claims 1,8, paragraph [0154] EP 3498370 A1 FR 3075072 A CN 110026216 A | |
| CN 105413670 A | 23 Mar. 2016 | (Family: none) | |
| WO 2014/046118 A1 | 27 Mar. 2014 | US 2015/0202597 A1 whole document CN 104640627 A KR 10-2015-0058325 A | |
| CN 106582603 A | 26 Apr. 2017 | (Family: none) | |
| CN 107118069 A | 01 Sep. 2017 | (Family: none) | |
| JP 2013-522271 A | 13 Jun 2013 | US 2013/0204057 A1 whole document WO 2011/113836 A1 EP 2366682 A1 CN 102892729 A KR 10-2012-0128685 A | |

Form PCT/ISA/210 (patent family annex) (January 2015)

**INTERNATIONAL SEARCH REPORT**
Information on patent family members

International application No.

PCT/JP2021/012965

| Patent Documents referred in the Report | Publication Date | Patent Family | Publication Date |
|---|---|---|---|
| JP 2017-537781 A | 21 Dec. 2017 | US 2017/0333876 A1 whole document WO 2016/086781 A1 EP 3228384 A1 CN 105709715 A KR 10-2017-0093157 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020064548 A

- WO 2015170686 A